# EUROPEAN PATENT SPECIFICATION

(11) **EP 3 628 292 B1**
(45) Date of publication and mention of the grant of the patent: **18.12.2024**
(21) Application number: 18196825.6
(22) Date of filing: 26.09.2018
(51) Int. Cl.: A61F 13/472, A61F 13/474, A61F 13/15, A61F 13/47

(54) **ADAPTABLE ABSORBENT ARTICLE**
ANPASSBARER SAUGFÄHIGER ARTIKEL
ARTICLE ABSORBANT ADAPTABLE

(43) Date of publication of application: 01.04.2020
(73) Proprietor: The Procter & Gamble Company, Cincinnati, OH 45202 (US)
(72) Inventor: Denti, Federica, 65824 Schwalbach am Taunus (DE); Chernenkaya, Alisa, 65824 Schwalbach am Taunus (DE); Meisl, Sabine, 65824 Schwalbach am Taunus (DE)
(74) Representative: P&G Patent Germany

(56) References cited:
- EP-A1- 1 994 918
- EP-A1- 2 011 460
- EP-A2- 1 757 257
- US-A1- 2002 077 613
- UNKNOWN: "Carefree Flexiform Slipeinlagen für Slip & Tanga "fresh"", 2 November 2016 (2016-11-02), XP055564458, Retrieved from the Internet <URL:https://www.pinkmelon.de/test/pflege-2/damenhygiene/slipeinlagen/produkt/carefree-flexiform-slipeinlagen-fuer-slip-tanga-fresh> [retrieved on 20190305]
- ANONYMOUS: "Carefree Flexiform Fresh Breathable Pantyliners (Regular & Tanga) 2 Packs x 30 ... by Carefree", 28 October 2015 (2015-10-28), XP055564459, Retrieved from the Internet <URL:https://www.amazon.de/Carefree-Flexiform-Breathable-Pantyliners-Regular/dp/B01LXXWU46> [retrieved on 20190305]

## Description

### FIELD OF THE INVENTION

The present invention is directed to absorbent articles having a chassis that is symmetrical about the transverse axis and an absorbent core that is asymmetrical about the transverse axis.

### BACKGROUND OF THE INVENTION

Sanitary napkins are used by women principally during their menstrual periods to receive and contain menses and other vaginal discharges to protect their garments from soiling. Similar products also exist for use when suffering from incontinence.

Pantiliners serve much the same purpose as sanitary napkins; the distinction is primarily in the overall size, including thickness. Pantiliners are generally less bulky and are designed to protect the user's clothing from relatively small quantities of vaginal discharges.

Many women have developed the habit of wearing an absorbent device between their menstrual periods to protect their clothing from any vaginal discharges, including light urinary discharge, and sometimes anal discharge. Because a sanitary napkin is generally too bulky for constant wear, such users generally utilize pantiliners.

As pantiliners may be worn by a user on a daily basis, users prefer for the pantiliner to have a "barely there" feel, while still providing adequate protection for their needs. Furthermore, as the shape and style of underwear worn by a user changes on a daily basis, there is a need for pantiliners intended to be worn every day to be suitably adaptable.

EP1994918A1 discloses pantiliners comprising a printed pattern visible through at least a portion of topsheet outside the core area.

Commercially available pantiliners are available for use with so called tanga underwear that tends to have a crotch with a wider front and narrower rear portion. Tanga underwear, due to its nature, also tends to conform more to the shape of a user's body than traditional underwear. To ensure discretion during use with tanga underwear, existing commercially available pantiliners are typically either shaped to fit (i.e., having a wider front and narrower rear) or they may be adaptable - typically having an asymmetrical core that has a wider front and narrower rear and a topsheet and backsheet that extend beyond and around the core in a symmetrical dogbone shape. However, in both cases this usually results in a smaller surface area for the absorbent core. Thus, there exists a trade off between comfort and protection when designing pantiliners for use with tanga underwear. Either the core can be made thicker, which tends to decrease the stiffness and flexibility of the liner, and as a result the overall comfort levels, or the pantiliner will have less absorbent capacity than required.

Furthermore, today there are no adaptable pantiliners on the market that are truly suitable for use with both normal underwear and tanga underwear or any shape or size of underwear between the two. For example, several commercially available adaptable pantiliners are provided. However, these pantiliners are found in general to be too stiff and non-compliant with movement of a user's panties. Furthermore, the stiffness, particularly in the core-free areas, means that it is hard to fold over the core free edges. To account for this, the current commercially available adaptable pantiliners are provided with score lines either side of the tapered core, thus enabling users to fold the pantiliners back around their panties along only these specific lines.

Thus, there exists a need to provide pantiliners that have the desired absorption levels, while still being comfortable and having the necessary adaptability.

### SUMMARY OF EXEMPLARY FORMS

The present invention is directed to absorbent articles, for example, pantiliners, for use with panties having varying crotch dimensions and oftentimes, an asymmetrical shape, for example, tanga panties, high and low cut panties.

As indicated in claim 1, the present invention is for an absorbent article having a longitudinal axis and a transverse axis, the article comprising a topsheet, a backsheet, wherein the topsheet and the backsheet together form a chassis of the absorbent article, said chassis being symmetrical about the transverse axis, an absorbent core disposed between the topsheet and the backsheet, wherein a perimeter of the chassis extends beyond the surface area of the absorbent core, and wherein the absorbent core is asymmetrical about the transverse axis, wherein less than 50% of the surface area of the absorbent article is mechanically compressed, and wherein the absorbent article has an in-use flexibility of less than 170mN, preferably less than 130 mN.

Further preferred features of the invention are indicated in the dependent claims.

BRIEF DESCRIPTION OF THE DRAWINGS

The following detailed description of specific forms of the present invention can be best understood when read in conjunction with the drawings enclosed herewith.
Figure 1 is a view from above of an absorbent article as described herein;
Figure 2 is an exploded view of the absorbent article of Figure 1 illustrating the respective layers;
Figure 3 is a further exploded view of the absorbent article of Figure 1 showing the layers of Figure 2 and additional adhesive layers;
Figure 4 is a simplistic top view of the absorbent article shown in Figure 1 illustrating areas for which a cross-section through the layers is shown in Figures 5A, 5B and 5C;
Figures 5A, 5B and 5C are cross-sectional view of the absorbent article shown in Figure 4;
Figure 6 is a top view of the absorbent article highlighting embossed regions of the absorbent core;
Figures 7A - 7D show the adaptable nature of the absorbent article described herein;
Figures 8A and 8B show schematically the steps of cutting the absorbent core;
Figure 9 shows schematically the manufacturing process of the absorbent article described herein;
Figure 10A and 10B show schematically the respective processes for embossing and printing;
Figure 11 shows schematically the test equipment for measuring in-use flexibility.

### DETAILED DESCRIPTION

The present invention is generally directed to feminine hygiene articles, which are known to be absorbent articles worn externally by women, usually to absorb vaginal discharge and/or urine leakage. The term feminine hygiene articles includes such articles commonly referred to as pads, pantiliners, liners, sanitary napkins, sanitary towels, or interlabial devices. These articles are typically held in place adjacent the user's pubic region by the user's undergarment, and may be affixed thereto via adhesive or other joining means.

As used herein, the term "nonwoven web" refers to a web having a structure of individual fibers or threads which are interlaid, but not in a repeating pattern as in a woven or knitted fabric, which do not typically have randomly oriented fibers. The basis weight of nonwoven fabrics is usually expressed in grams per square meter (gsm). The basis weight of a nonwoven web/laminate is the combined basis weight of the constituent layers and any other added components. Fiber diameters are usually expressed in microns, fiber size can also be expressed in denier, which is a unit of weight per length of fiber.

As used herein, "spunbond" fibers" refer to small diameter fibers which are formed by extruding molten thermoplastic material as filaments from a plurality of fine, usually circular capillaries of a spinneret. Spunbond fibers are generally not tacky when they are deposited on a collecting surface. Spunbond fibers are generally continuous and have average diameters (from a sample of at least 10) larger than 7 microns, and more particularly, between about 8 and 40 microns.

As used herein "philic" and "phobic" have meanings as well established in the art with respect to the contact angle of a referenced liquid on the surface of a material. Thus, a material having a liquid contact angle of greater than about 75 degrees is considered phobic, and a material having a liquid contact angle of less than about 75 degrees is considered philic.

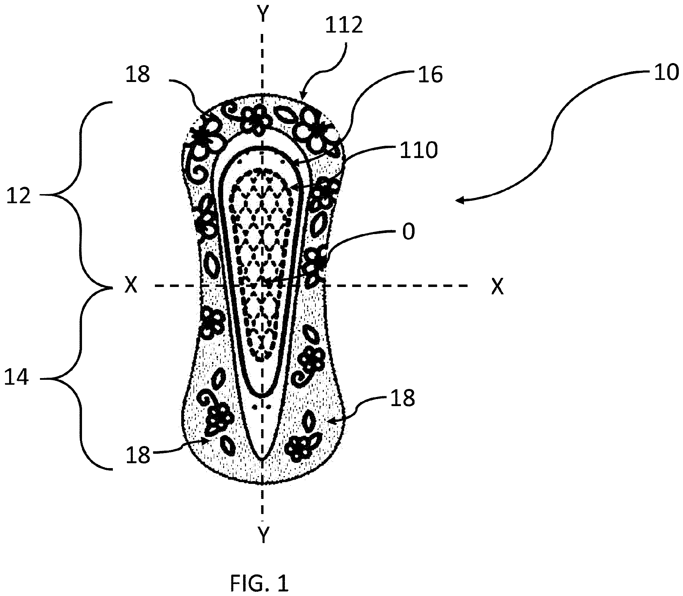

Figure 1 shows a top view of the body-facing surface of an exemplary absorbent article 10. The particular embodiment shown is an example of a pantiliner (also sometimes designated as a "liner" or "panty-liner"), but the present invention is not limited thereby. Such absorbent articles normally have a generally flat body-facing surface but are generally flexible to adapt to the user's anatomy and movements. They can also be folded and wrapped prior to packaging, for example, to facilitate more hygienic transport of individual articles and/or to reduce the size of packaging.

The pantiliner shown in Figure 1 has a longitudinal axis Y-Y and a transverse axis X-X intersecting at a centrepoint 0 of the pantiliner. A front region 12 is provided on one side of the transverse axis and a rear region 14 is provided on the other side of the transverse axis. While the pantiliner may have any shape known in the art, a preferred shape is generally "hour-glass" shaped, tapering inwardly from a relatively greater transverse width in the front and rear regions to a relatively smaller transverse width at the middle region. Pantiliners may also be provided with lateral extensions known commonly in the art as "flaps" or "wings" (not shown) that are typically intended to extend over and cover panty elastic in the crotch region of the user's undergarment.

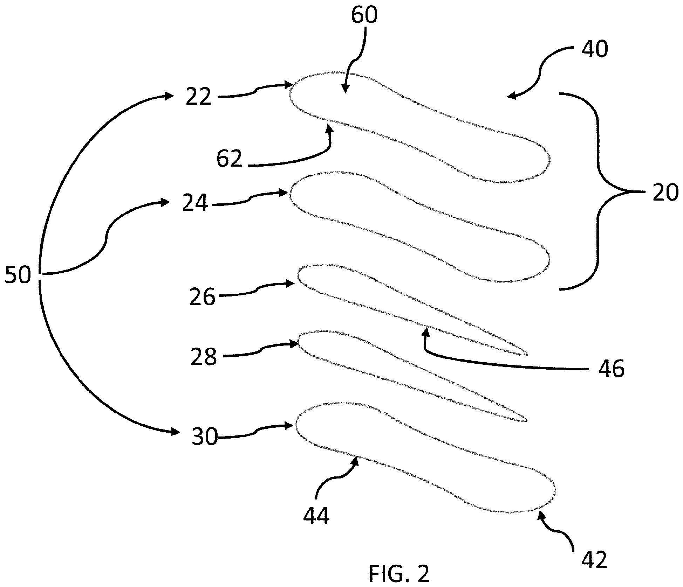

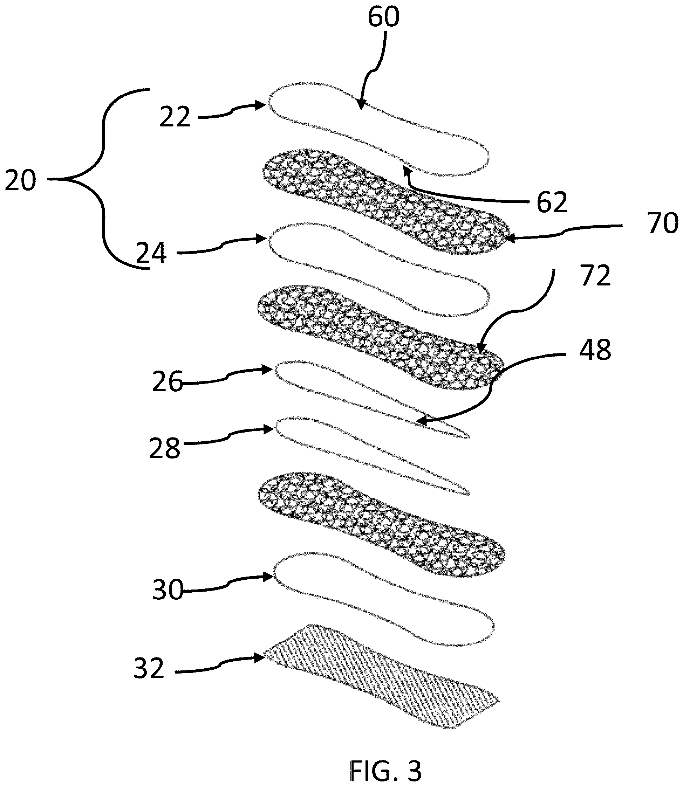

Figure 2 represents the article of Figure 1 in an exploded view showing, from top to bottom, the following layers: a laminated topsheet 20 comprising a first topsheet 22 and a secondary topsheet 24, an absorbent core 26, a liquid impervious patch 28 and a backsheet 30. A releaseable cover 32 which may be used to cover an adhesive material on a garment facing surface of the backsheet is shown in Figure 3. The "top" 40 of the article is defined herein as the surface of the article oriented toward the user's body, and the "bottom" 42 is defined herein as the opposite surface of the article, i.e., the surface that will generally contact the woman's undergarment.

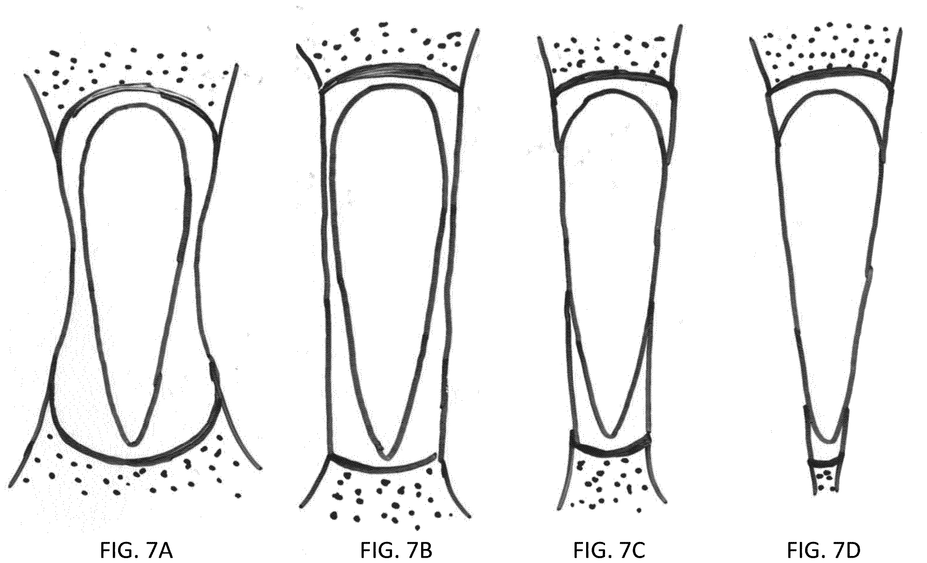

The chassis 50 of the pantiliner, comprising the topsheet (or laminated topsheet where applicable) and backsheet is symmetrical about the longitudinal and transverse axes. The absorbent core is symmetrical about the longitudinal axis, but asymmetrical about the transverse axis, with more than 50% of the core being located in the front region of the pantiliner. Thus, the pantiliner is provided with a core region 16 and one or more core free regions 18. The pantiliner is intended to be adaptable from a first form (as shown schematically in Figure 7A) where the pantiliner is not folded in any way and where pantiliner has the shape of the topsheet and backsheet, i.e., it is symmetrical about both the longitudinal axis and the transverse axis, and a second form for use with different undergarments, for example, tanga panties (as shown schematically in Figure 7B, C and D), where at least part of the core free region of the absorbent article may be folded and adhered to an underside of the undergarment.

### Topsheet

The topsheet 20 has a body facing surface 60 and a garment facing surface 62. Typically, the garment facing surface 62 is located adjacent the absorbent core 26 or any other layers provided between the absorbent core 26 and the topsheet. Any conventional materials may be used to form a topsheet that may be used in the absorbent articles described herein. Topsheets are typically formed of soft, smooth, compliant, porous materials which are comfortable against human skin and through which fluids such as urine or vaginal discharge, such as menstrual fluids, may pass. A suitable topsheet can be formed of various materials such as woven and nonwoven materials; aperture film materials including aperture formed thermoplastic films, aperture plastic films, and fiber-entangled aperture films, hydro-formed thermoplastic films; porous foams, reticulated foams; reticulated thermoplastic films, thermoplastic scrims, or combinations thereof. Nonlimiting examples of woven and nonwoven materials suitable for use as the primary topsheet include fibrous materials made from natural fibers, modified natural fibers, synthetic fibers, or combinations there.

The topsheet may also be a laminated topsheet as shown in Figure 2. Such a laminated topsheet may be formed of two layers of different, but complimentary, materials selected from those listed above that are adhered to one another to form a unitary layer.

For example, in a laminated topsheet, the primary topsheet that is located at the top of the pantiliner and comes into contact with a user's body, may be designed to allow liquids to pass through quickly and easily, while still providing a comfortable skin contact surface. The secondary topsheet may have good fluid acquisition properties. For example, the primary topsheet may be a phobic nonwoven material, for example a spun bonded polypropylene or multi/bi-component polyethylene and polypropylene nonwoven having soft properties and rapid strike through and good rewet properties and the secondary topsheet may be a philic or semi-philic material, such as a carded air through bonded PET material having a uniform, open structure, good resilience, rapid fluid strike-through and fluid distribution. The combination of the two materials may provide for more effective and rapid fluid acquisition from the body-facing surface of the topsheet to the core and minimum rewet, while still providing a comfortable contact surface for the user. The eventual choice of materials may depend on the materials that are readily available and cost effective, while still providing the above described benefits.

Preferably, the secondary topsheet has the same shape and size as the topsheet such that it extends to the edge of the chassis, as shown best in Figures 2 and 3. The primary and secondary topsheet may be laminated together prior to cutting to shape. In such embodiments, the primary and secondary topsheets are adhered together across their entire footprints (as indicated by the layer of glue 70 shown in Figure 3). Preferably, the laminated topsheet is not itself embossed, crimped or in any other way mechanically compressed. Without being bound by theory, it is thought that mechanical compression, e.g., in the form of embossment, reduces the flexibility and resilience of the resultant laminated topsheet.

Preferably, the topsheet has a basis weight of between 15 gsm and 60 gsm, preferably between 25 gsm and 50 gsm, preferably between 35 gsm and 45 gsm. Where the topsheet is a laminated topsheet, the primary topsheet preferably has a basis weight of between 5 gsm and 25 gsm, 10 gsm and 20 gsm and preferably between 15 gsm and 18 gsm, preferably 17 gsm and the secondary topsheet has a basis weight of between 10 gsm to 40 gsm, preferably 20 gsm to 30 gsm and 22 gsm to 26 gsm, preferably 24 gsm.

The topsheet may comprise some form of printed pattern intended to be visible to a user from a body-facing surface of the absorbent article. The printed patterns may be used to communicate functional areas of the absorbent article to a consumer. Specifically in the context of adaptable pantiliners, this can help consumers correctly orient the pantiliner (particularly when the pantiliners are stored in a package in alternating directions) and to correctly position the pantiliner in the user's panties, ensuring the part of the pantiliner with greatest absorption properties are positioned in the area of the panty most likely to receive exudates. As described in more detail herein, the printed pattern is preferably applied to the garment-facing surface of the primary topsheet to avoid risk of transfer of ink to a user's body, while still having the required intensity to appropriately communicate to the consumer.

### Backsheet

The backsheet 30 is located at the bottom 42 of the absorbent article and provides a garment facing surface 44 of the absorbent article. The backsheet may or may not be breathable. The backsheet may be impervious, or substantially impervious, to liquids (e.g., urine) and may be manufactured from a thin plastic film, although other flexible liquid impervious materials may be used. As used herein, the term "flexible" refers to materials which are compliant and will readily conform to the general shape and contours of the human body. The backsheet may prevent, or at least inhibit, the exudates absorbed and contained in the absorbent core from wetting articles of clothing which contact the pantiliner, such as undergarments or panties. Alternatively, the backsheet may be a liquid permeable flexible layer formed of, for example, thin nonwoven material (for example a stretchable, spun laced, non-woven material having a basis weight of from about 30 g/m² to 40 g/m²), formed of polyethylene terephthalate or polypropylene fibers may be used, perforated plastic films, net material, liquid-permeable foam material or the like. In cases where the backsheet is liquid permeable, preferably a secondary liquid impermeable patch (as described below) is provided to minimize the chance of leakage.

The backsheet may be printed on the body facing surface or the garment facing surface. Furthermore, a garment facing surface of the backsheet typically has adhesive provided to it, for adhesion of the absorbent article to a user's undergarment. The placement and volume of the adhesive may be selected for optimal contact with a user's undergarment, dependent on the size, shape and thickness of the absorbent article. Preferably, the adhesive is applied across substantially the entire surface area of the garment facing surface 44 of the backsheet (not shown) such that the pantiliner effectively moves together with the undergarment to which it is attached.

### Absorbent Core

The absorbent core 26 is disposed between the topsheet and the backsheet. As used herein, the term "absorbent core" refers to a material or combination of materials suitable for absorbing, distributing, and storing fluids such as urine, blood, menses, and other body exudates.

The absorbent core may be made of any suitable liquid-absorbent material. Non-limiting examples of liquid-absorbent materials suitable for use as the absorbent core include comminuted wood pulp which is generally referred to as airfelt; creped cellulose wadding; absorbent gelling materials including superabsorbent polymers such as hydrogel-forming polymeric gelling agents; chemically stiffened, modified, or cross-linked cellulose fibers, meltblown polymers including coform; synthetic fibers including crimped polyester fibers; tissue including tissue wraps and tissue laminates; capillary channel fibers; absorbent foams; absorbent sponges; synthetic staple fibers; peat moss; or any equivalent material; or combinations thereof.

The absorbent core has a relatively smaller surface area than that of the topsheet and backsheet. As shown in Figure 1, the absorbent core described herein is symmetrical about the longitudinal axis, but asymmetrical about the transverse axis, with a greater percentage of the absorbent core positioned in the front region of the absorbent article. Preferably, at least 50%, 52% or 54% of the absorbent core is positioned in the front region of the absorbent article.

Preferably, the absorbent core has a basis weight of between 100 gsm to 170 gsm, preferably 120 gsm to 150 gsm, preferably 130 gsm to 140 gsm.

### PE Patch

The liquid impermeable patch 28 is a layer of impermeable material that may be provided between the backsheet 30 and the garment-facing surface 46 of the absorbent core 26. A liquid impermeable patch may preferably be used in instances where the backsheet is permeable to provide an extra barrier against potential leakage of exudates from the pantiliner, while still allowing air to flow around the perimeter of the absorbent core. The liquid impermeable patch may be formed of materials such as thin plastic films and coatings of resin, wax, liquid tight adhesive or the like.

### Absorbent Article

Pantiliners such as those described herein have an overall chassis, comprised of the topsheet and backsheet with at least an absorbent core disposed between the topsheet and backsheet. The chassis has the overall shape of the pantiliner and is generally symmetrical about both the longitudinal and transverse axes. As shown in Figure 1, 2 and 3, the chassis may be dogbone shape, such that it is wider at the front and rear regions and narrower at a central region, although other known shapes may also be used. As pantiliners are intended for regular, perhaps everyday, use and for light flow or as a back-up to other adult incontinence or feminine hygiene devices, they are typically small in surface area, thin and light. Thus, the chassis preferably has a length of less than 250 mm, preferably less than 230 mm, preferably less than 200 mm, although it will be appreciated that different lengths may be chosen for different users, body shapes or undergarments.

As shown in the exploded view of Figure 3, the topsheet 20 (in this case laminated, although it may be a single layer) is adhered to the body facing surface 48 of the core and to the area of the backsheet surrounding the core using hotmelt adhesive 72. Preferably, the adhesive is provided around the entire periphery of the core and extends to the periphery of the chassis such that any other form of fixing (e.g., crimping) is not required. Thus, preferably, an absorbent article as described herein does not have a crimped edge. Without being bound by theory, it is thought that as adhesive is provided around the edge of the core, this minimizes the chance of leakage from the sides of the core out of the pantiliner, as the adhesive provides an extra barrier. Furthermore, as crimping around the edges of the liner is not required, this improves the overall in-use comfort and enables better overall stiffness and extensibility properties of the pantiliner.

As described above, an absorbent core and, optionally, an impermeable patch are provided between the topsheet and the backsheet. The pantiliner has a thickness through the core of between 0.5 mm and 2.0 mm, preferably between 0.75 mm and 1.75 mm, preferably between 1.0 mm and 1.5 mm. The pantiliner further has a thickness in the core free regions of between 0.1 mm and 0.7 mm, preferably between 0.25 mm and 0.65 mm, preferably between 0.35 mm and 0.6 mm, preferably between 0.45 mm and 0.55 mm, preferably about 0.5 mm.

The pantiliners described herein have specifically been designed for use with both conventional and non-conventional shaped underwear and particularly for daily use for adult incontinence. There is a continual tradeoff between absorbency needs and comfort and softness. Thus, for the specific market of pantiliners, it is preferred that the pantiliner described herein has an overall absorbent capacity of at least 3g, 5g, 7g, 8g or 9g and preferably less than 20g, 15g, 13g or 12g.

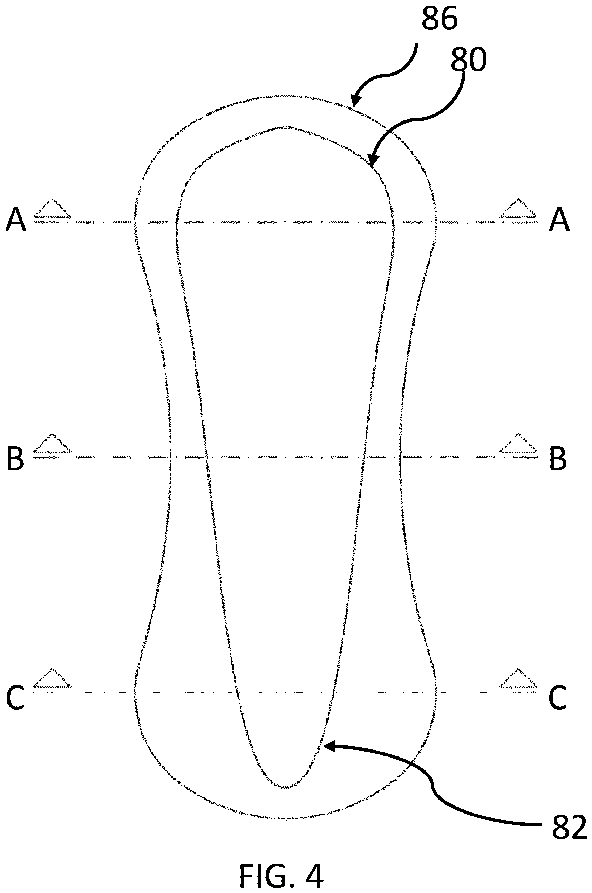

The absorbent core has a front end 80 and a rear end 82, as shown in Figure 4. The front end of the core is rounded and is substantially parallel to a front end 86 of the chassis. Preferably, a periphery of the front end of the core is no more than 7 mm, preferably 6.5 mm from a periphery of the chassis. Sides of the core are tapered from the front rounded end of the absorbent core to the rear end of the core, where the sides come together at an angle of between 3.5 ° and 9.5 °, preferably between 5° and 8°, preferably between 6° and 7°. Preferably, the rear end of the core is additionally rounded to enable folding of the pantiliner around a variety of different panty shapes and sizes. Without being bound by theory, it is thought that the shape of core shown provides balance between absorption coverage and area around the core that can easily be folded around undergarments.

The core occupies at least 50%, 51% or 52% of the total surface area of the chassis. Preferably, at least 50%, 53%, 55%, 57% or 60% of the core is provided in the front region of the chassis/pantiliner. The asymmetrical core described herein is designed to cover more of the surface area of the chassis than previously known adaptable pantiliners. In the example described herein, maximum absorbency is provided in the area where it is most needed (i.e., the front of the pantiliner). In the rear of the pantiliner, the core free area, that is more suitable for bending for adaptation to different panties, is maximized.

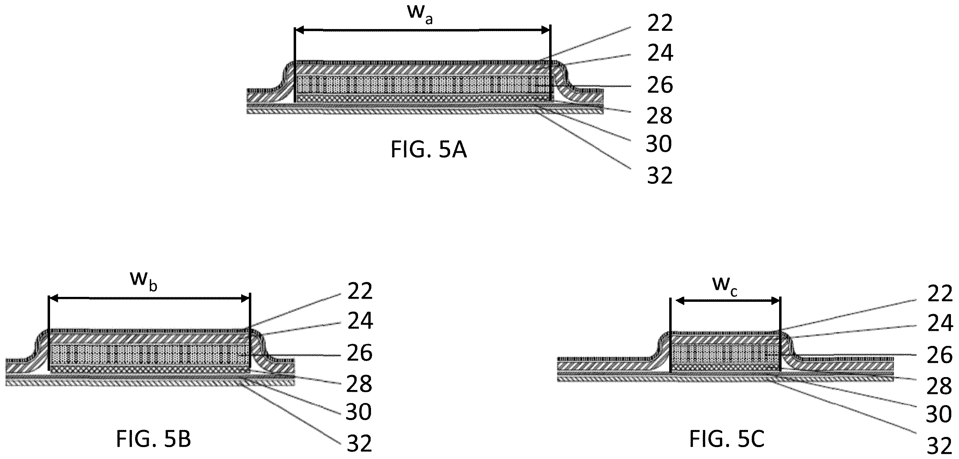

Thus, in the front region of the absorbent article, the absorbent core occupies at least 60% of the surface area between the topsheet and the backsheet, preferably at least 65% whereas in the rear region, the absorbent core occupies less than 50% of the surface area between the topsheet and the backsheet, preferably less than 45%. Cross-sections through the widest points of the front and rear regions and along the central transverse axis are shown in Figures 5A, 5B and 5C from where it can be seen that the width of the absorbent core varies relative to the width of the chassis. At the widest point of the rear region of the chassis, the width of the absorbent core is less than 40% of the chassis width. For example, in the example shown in Figure 5A, the chassis width may be between 60 and 65 mm, and the core width at the same cross-sectional point is between 19 mm and 21 mm. The reduced width of core at this point provides a user with a significant degree of freedom within which to fold the core free regions of the pantiliner back over panties.

The absorbent article described herein preferably has a stiffness in the core region of between 0.5 g*cm and 4 g*cm, preferably between 1.5 g*cm and 3.5 g*cm, preferably between 2.0 g*cm and 3.0 g*cm, preferably between 2.25 g*cm and 2.75 g*cm. The stiffness of the core provides for better comfort to a user of the pantiliner during use. In his respect, tanga panties, in particular, conform more to a user's body than standard underwear. Thus, providing a pantiliner with reduced stiffness throughout the core area increases the comfort level experienced by a user. Furthermore, in the current example, this additional comfort is provided without a significant reduction in overall absorbency provided by the product. Thus, the product performs in a comparable way vs other known adaptable pantiliners, but with more comfort.

Preferably, the absorbent article described herein has an in-use flexibility measurement of between 85 mN and 170mN, preferably between 90 mN and 150 mN, preferably between 100 mN and 130 mN, preferably between 105 mN and 120 mN. The in-use flexibility test (described hereafter) provides an indication of the flexibility of the absorbent article when in adapted form, i.e., when at least part of the core free region is folded back around the perimeter of a user's panties. This gives a "real-life" indication of the comfort that a user may experience when using an absorbent article as described herein.

Without being bound by theory, it is thought that the core stiffness and in-use flexibility is attributable at least in part to the thickness of the core layer and to the area of the core that is embossed, or otherwise mechanically compressed.

Furthermore, the absorbent article preferably has a stiffness in the core free area of between 0.01 g*cm and 0.4 g*cm, preferably between 0.025 g*cm and 0.3 g*cm, preferably between 0.05 g*cm and 0.2 g*cm, preferably between 0.075 g*cm and 0.15 g*cm. The stiffness in the core area determines to a large degree the ease with which a user may be able to fold over the core-free regions of the pantiliner around their panties. By having reduced stiffness in this area, the described pantiliner is easier to fold over, therefor being genuinely adaptable around any form or shape of underwear and without having any uncomfortable folding points. Without being bound by theory, it is thought that the stiffness of the present described absorbent article is lower than that of other commercially available adaptable pantiliners as the present absorbent article has much less mechanical compression across the entire product and particularly in the core free areas. Mechanical compression by nature forms dense areas of fibers which may increase stiffness of the absorbent article.

Thus, preferably less than 50%, preferably less than 40%, and preferably less than 30%, preferably less than 20%, preferably less than 15% of the total surface area of the absorbent article (i.e., the surface area of the chassis) is mechanically compressed. "Mechanically compressed" as used herein includes any known method of compression, including but not limited to: scoring, stitching, embossing, crimping or perforating. Preferably less than 50%, preferably less than 40%, preferably less than 30% of the surface area of the core is mechanically compressed. Preferably less than 50%, preferably less than 40%, preferably less than 30%, preferably less than 20%, preferably less than 10% of the core-free area is mechanically compressed. The core-free area may be substantially free of any kind of mechanical compression, for example, crimping or embossing.

Adhesive may be applied to the garment facing surface 44 of the backsheet for adhesion of the absorbent article to a user's undergarment. The adhesive may be any adhesive known in the art. As a non-limiting example, pressure sensitive adhesive strips, swirls, or waves may be applied to help maintain the absorbent article in place. As used herein, the term pressure-sensitive adhesive refers to any releasable adhesive or releasable tenacious means. Suitable adhesive compositions, include, for example, water-based pressure-sensitive adhesives such as acrylate adhesives. Alternatively, the adhesive composition may include rapid setting thermoplastic "hot melt", rubber adhesives, two-sided adhesive tape, and the like.

The absorbent article may further comprise a paper release strip 32 (shown in Figure 3), which has been coated on one side, to be applied to the garment facing surface 44 of the backsheet. The coating on the paper release strip, which may be silicone, reduces the adherency to the adhesive of the coated side of the release paper. The release strip can be formed from any suitable sheetlike material which, when coated, adheres with sufficient tenacity to the adhesive to remain in place prior to use but which can be readily removed when the absorbent article is to be used.

### Absorbency Indicator

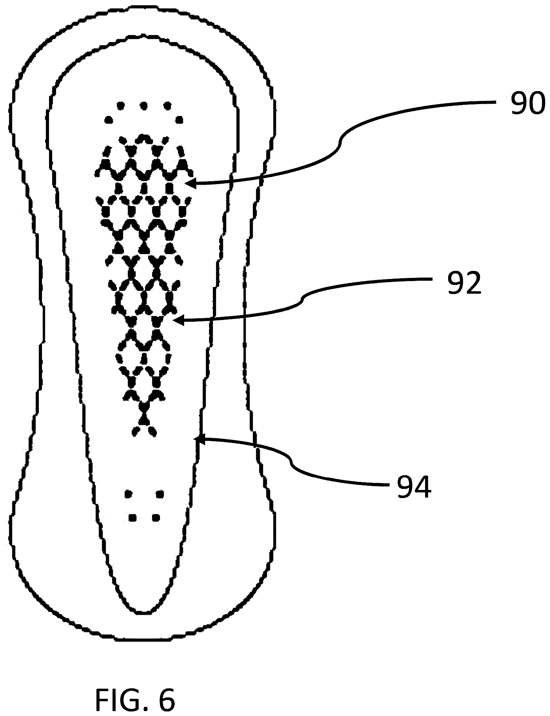

Pantiliners as described herein are provided with one or more absorbency indicators. The absorbency indicators help communicate to the consumer different functions associated with the pantiliner. For example, in some embodiments, the absorbency indicator is an embossed pattern 90 (shown in Figure 6) located within the core region of the pantiliner that communicates to a user where the maximum absorptive capacity of the pantiliner can be found. Additionally and/or alternatively, printed patterns may be used in the same way as an embossed pattern - for example, with a printed pattern provided within the core region 26. Printed patterns may also be used to provide an indication to the user of the adaptability of the pantiliner, for example, by applying a greater density of printed pattern in the area where the core is provided and less in the core-free areas. It will be appreciated that the absorbency indicator may take any form known in the art that can connote functionality of the pantiliner to a user.

One example of an absorbency indicator is shown in Figure 6 as an embossed pattern 90, provided in the core region 26 of the pantiliner. Preferably, the topsheet 20 is embossed together with the absorbent core, such that the embossed pattern extends through at least the topsheet and absorbent core. As shown in Figure 6, the shape of the embossed pattern is substantially the same as the asymmetric absorbent core and the periphery of the embossed pattern 92 is substantially parallel to the periphery 94 of the absorbent core. Preferably the periphery of the embossed pattern is positioned inward of the periphery of the absorbent core.

Preferably, the embossed pattern is provided on between 10% and 50% of the surface area of the absorbent core. Furthermore, as with the absorbent core, preferably, between 50% and 90%, 55% and 85%, 60% and 80% or 65% and 75% of the embossed pattern is provided in the front region of the absorbent article. Preferably, at least 90%, 93%, 95%, 97% or 100% of the embossed pattern is provided on the absorbent core, with substantially no embossing or other mechanical compression on the core free areas of the chassis.

Without being bound by theory, it is thought that the positioning and size of the embossed pattern serves multiple purposes: to provide a functional indication to a user of the area of the absorbent article with maximum absorptive capacity, to provide enhanced absorption via liquid transfer from the topsheet to the absorbent core in the area of the absorbent article with the greatest volume of absorbent core.

Furthermore, by limiting the embossed pattern to the area of the pantiliner where it is specifically required for functional communication or efficacy reasons, the overall area of the pantiliner that is embossed is reduced. This contributes to an overall softer feeling, more stretchable and less stiff material compared with pantiliners that feature embossing over the entire surface area as described above.

A printed pattern that is visible from the body facing surface of the absorbent article may also be provided, as best seen in Figure 1. The pattern may be printed on any surface of any of the layers of the absorbent article provided it is visible to a user, however, preferably, the pattern is printed on the garment facing surface of the topsheet, and where a laminated topsheet is provided, preferably, the garment facing surface of the primary topsheet.

As can be seen in the embodiment of Figure 1, two printed patterns are provided. A first printed pattern 110 is provided wholly in the core region, and has a shape substantially parallel to the perimeter of both the embossed pattern and the absorbent core. A second printed pattern 112 is provided on the periphery of the absorbent article and partially overlaps the absorbent core. A greater percentage of the second printed pattern is provided in the front region of the absorbent article, where the absorbent core is not present. Preferably, more than 50%, preferably more than 55% of the second printed pattern is provided in the front region of the absorbent article. Without being bound by theory, the first printed pattern provides a visual queue to a user about the orientation of the absorbent article and the area of maximum absorption, thereby enabling the user to correctly position the absorbent article. By focusing the majority of the second pattern in the front part of the absorbent article, it is easier for the user to see the core free areas, particularly in the rear region of the pantiliner, thus enabling a better appreciation of where the pantiliner may be folded.

### Manufacturing Method

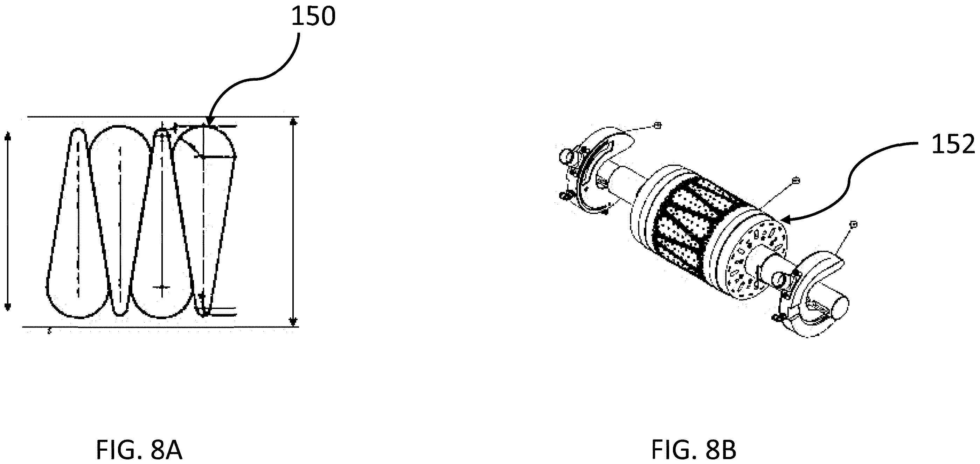

Figures 8A and 8B shows a nested configuration of cross-directionally oriented absorbent cores 150. A continuous web of core material 126 is delivered in a machine direction to a rotary cutter 152, and oppositely oriented asymmetrical cores are cut from the continuous web of core materials. In embodiments where the pantiliner comprises an impermeable patch, a web of impermeable material may be combined with the absorbent core material prior to first cutting such that the absorbent core and impermeable patch have the same shape and size. Although, it will be appreciated that the impermeable patch may be a different shape and size to the absorbent core.

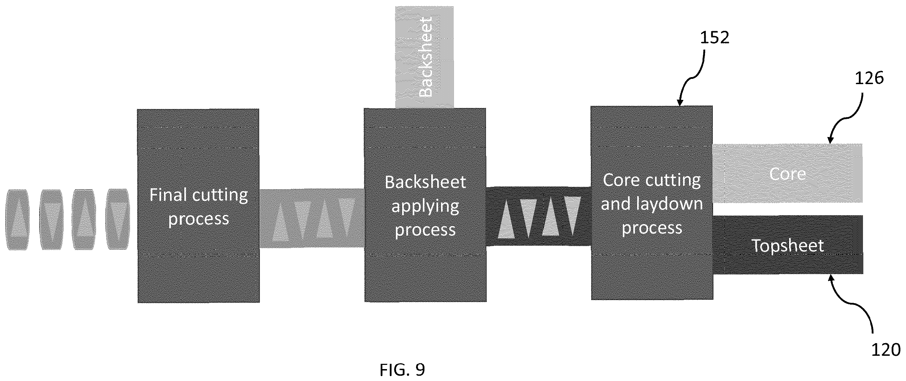

The individual absorbent cores form a repeating nested pattern of generally identically shaped and oppositely oriented absorbent cores. As shown schematically in Figure 9, the body facing surfaces of the absorbent cores (together with the impermeable patch if applicable) are then laid down onto a continuous web of topsheet material 120 in the direction and order in which they have been cut with a space between them. Where the topsheet is a laminated topsheet, the primary and secondary topsheet layers may be combined together to form a continuous web of laminated topsheet prior to entry into the final assembly process, with the primary topsheet facing the belt and the secondary topsheet receiving the absorbent cores. A continuous web of backsheet material 130 is then applied on the garment facing surfaces of the absorbent core (or, where there is an impermeable patch, onto the garment facing surface of the impermeable patches) and the final product form is cut.

The resultant pantiliners are output from the system in alternating directions about the transverse axis, thus alternate pantiliners have opposite facing front and rear regions, as determined by the positioning of the absorbent core.

The present manufacturing system minimizes waste of material and enhances speed and efficiency of manufacturing. In this respect, alternate absorbent cores share boundary walls as they are cut in the first step, thus no material between adjacent absorbent cores is lost. Only a very small amount of absorbent core material is wasted at the top and bottom of each absorbent core. Furthermore, given the nested cutting of adjacent absorbent cores, it is possible to cut fully tapered absorbent cores without wasting considerable material. This is contrary to absorbent cores that are cut parallel to the machine direction where all material outside the tapered edges is wasted. Likewise, as the absorbent cores are laid down in the same direction between the continuous webs of topsheet and backsheet, adjacent pantiliners in the widest parts of the front and rear regions share boundary walls, such that waste between adjacent pantiliners is limited to the central and top and bottom of adjacent pantiliners. Furthermore, given the rounded shape of the front region of the absorbent core, it is possible to cover an overall larger surface area of the front region of the pantiliner with absorbent core material, thus providing enhanced absorbent coverage in the area of the pantiliner where it is really needed.

Without being bound by theory, it is also considered that the stretchability of the pantiliner in the longitudinal direction is enhanced as a result of the direction of manufacture. In this respect, the greatest area of flexibility required by users is in the longitudinal direction, as the greatest variation, particularly when sitting down/standing, is seen. As mentioned above, this is particularly true in the absence of embossing of the laminated topsheet.

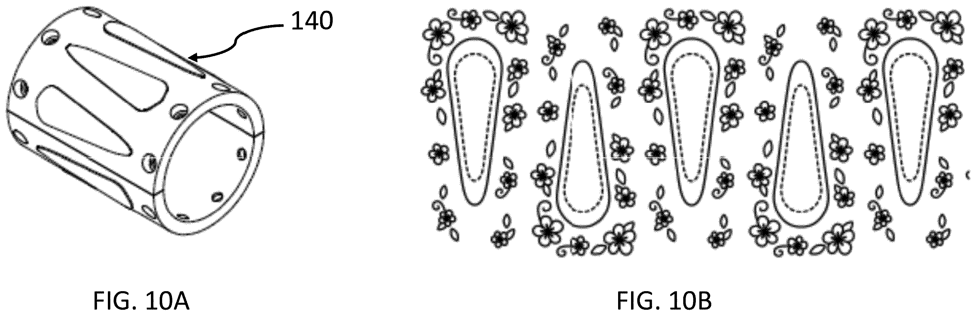

Preferably, the garment facing surface of the topsheet is printed prior to laying down of the absorbent core or, where a laminated topsheet is provided, the garment facing surface of the primary topsheet is printed prior to lamination with the secondary topsheet. By printing on the surface of the topsheet that is not facing the body of the user, the ink will not come into direct contact with a user's skin. Furthermore, by printing on the garment facing surface of the primary topsheet (rather than the secondary topsheet), the intensity of ink seen from the body facing surface of the topsheet is greater compared with the intensity of ink as seen through either a thicker single topsheet or the combined laminated topsheet. It will be appreciated that various types of printing processes may be used to create the patterns disclosed herein. For example, in some embodiments, flexography may be used. In particular, flexography may utilize printing plates made of rubber or plastic with a slightly raised image thereon. The inked plates are rotated on a cylinder which transfers the image to the sheet. As can be seen in Figure 10B, for the pantiliner described herein, where adjacent articles are oriented in the opposite direction, alternate printing plates on a cylinder are oriented in the opposite direction relative to the transverse axis. This ensures that the orientation of the printed pattern matches that of the asymmetrical core. Flexography may be a relatively high-speed print process that uses fast-drying inks. Other embodiments may utilize gravure printing. More particularly, gravure printing utilizes an image etched on the surface of a metal plate. The etched area is filled with ink and the plate is rotated on a cylinder that transfers the image to the sheet.

Embossing of the pantiliner preferably takes place after the core has been laydown on the topsheet, but prior to laydown of the backsheet and prior to cutting of the individual pantiliners. The embossing can be achieved with standard techniques such as thermal bond, ultrasonic bond and/or pressure. An example of a suitable process is thermal bonding wherein the layers are passed through two steel rolls where one is engraved with the visual pattern and the other is flat. In certain embodiments, one or both of the rolls are warmed to temperature suitable to at least partially melt one or more layer (typical range from 90 to 170 degrees C).

The embossing roll 140 (shown in Figure 10A) may be engraved using conventional techniques such as machine tooling for most embossing patterns, but it may be desirable to use acid etching or laser engraving to provide a finer engraving and thus a finer embossed pattern. It may be desirable that the embossed pattern comprises relatively thin embossing features, much thinner than the embossed channels previously disclosed in the art, such as in WO2003/006818. Thin embossing features may provide a generally feminine and delicate look to the article. The embossing tool should therefore be capable of high definition embossing, in particular with a resolution (minimum thickness of the embossed lines) of less than about 0.75 mm, in particular but not limited to between about 0.35 mm and about 0.60 mm. Given the alternating nature of adjacent pantiliners described herein, as shown in FIGURE 8C, the embossing roll preferably has alternately oriented embossing patterns pre-engraved into the mold.

### In Use

Figures 7A, 7B, 7C and 7D show schematically how the pantiliner described herein may be adapted for use with different shapes and sizes of panties, dependent on a user's needs. Figure 7A shows the pantiliner on conventional underwear having a narrow central region and a wider front and back region. With such panties, a pantiliner as described herein may be kept in the prefolded position. In this respect, the topsheet described herein is provided with a certain substance and opacity that reassures the user that even when the pantiliner is used in this position, it carries a certain degree of absorbency outside of the core area.

Figures 7B and 7C show schematically that the pantiliner described herein may be folded back over a user's panties in a number of different configurations dependent on the need. As there are no dedicated folding lines, a user has flexibility to choose how and where to fold back the core free region. Figure 7D shows schematically the pantiliner used in conjunction with known tanga panties where all but the core is adhered to the underside of the panty. The different parameters of thickness, stiffness and flexibility resulting from the construction of the pantiliner described herein contribute to the adaptability of the pantiliner (as shown below).

### Test Methods

### Samples

**Comparative Sample 1:** Carefree^{®} Flexiform Fresh (commercially available) Liner for Slip & Tanga.

### Inventive Sample 1:

Length: 151mm
Primary Topsheet: Nonwoven, spunbond polypropylene - basis weight = 17 gsm
Secondary Topsheet: Carded Airthrough nonwoven, polyethylene & polyethylene terephthalate bicomponent fibers - basis weight = 24 gsm
Absorbent core: airlaid core including superabsorbent particles - basis weight = 160 gsm
Liquid impermeable patch: polypropylene film - basis weight = 19 gsm
Backsheet: nonwoven spunbond polypropylene - basis weight = 18 gsm

Both comparative sample 1 and inventive sample 1 are intended for use as adaptable pantiliners having a centrally located core that is asymmetric about the transverse axis and a chassis that is symmetric about the longitudinal axis.

### Thickness

The thickness of a test sample is measured as the distance between a reference platform on which the test sample rests and a pressure foot that exerts a specified amount of pressure onto the test sample over a specified amount of time. All measurements are performed in a laboratory maintained at 23 °C ± 2 C° and 50% ± 2% relative humidity and test samples are conditioned in this environment for at least 2 hours prior to testing.

Thickness is measured with a manually-operated micrometer equipped with a pressure foot capable of exerting a steady pressure of 3.5 kPa ± 0.01 kPa onto the test sample. The manually-operated micrometer is a dead-weight type instrument with readings accurate to 0.01 mm. A suitable instrument is Mitutoyo Series 543 ID-C Digimatic, available from VWR International, or equivalent. The pressure foot is a flat ground circular movable face with a diameter that is smaller than the region of the test sample being measured, and capable of exerting the required pressure. A suitable pressure foot has a diameter of 6.85 mm, however a smaller or larger foot can be used depending on the size of the region being measured. The test sample is supported by a horizontal flat reference platform that is larger than and parallel to the surface of the pressure foot. The system is calibrated and operated per the manufacturer's instructions.

Obtain a test sample by removing it from any individual wrapper present. If folded, gently unfold it and smooth out any wrinkles. If present, remove the release paper to expose the adhesive on the garment facing side of the test sample. Lightly apply talcum powder to the adhesive on the backsheet to mitigate tackiness. The core region (depicted as 16 in Figure 1) is the area of the test sample that contains the primary absorbent core. The test location for the core region is preferably near the intersection of the longitudinal and transverse midpoints of the primary absorbent core in an area that is free from any folds. The core-free area (depicted as 18 in Figure 1) of the test sample, is in the rear region of the test sample and does not contain the primary absorbent core. In all cases, the test location being measured must be larger than the pressure foot and be free from any folds.

To measure thickness of the core region, first zero the micrometer against the horizontal flat reference platform. Place the test sample on the platform with the topsheet side of the test sample facing the pressure foot, and the test location centered below the pressure foot. Gently lower the pressure foot with a descent rate of 1.0 mm ± 0.1 mm per second until the full pressure is exerted onto the test sample. Wait 5 seconds and then record the thickness of the test sample's core region to the nearest 0.01 mm. In like fashion, repeat for a total of five replicate test samples. Calculate the arithmetic mean for thickness and report as Core Area Thickness to the nearest 0.01 mm.

In like fashion, measure the thickness of the Core-Free area for a total of five replicate test samples. Calculate the arithmetic mean for the thickness and report as Core-Free Area Thickness to the nearest 0.01 mm.

**TABLE 1:**

| **THICKNESS** | Comparative Sample 1 | Inventive Sample 1 |
|---|---|---|
| Core Area (mm) | 2.07 | 1.49 |
| Core-Free Area (mm) | 0.77 | 0.49 |

It can be seen in Table 1 that the pantiliner described herein is overall thinner than comparative pantiliners (Comparative Sample 1 is also an adaptable pantiliner targeting the same/similar user group). Thinner pantiliners (that preferably do not compromise on absorbent capacity or other comfort/efficacy vectors) are generally considered preferable to consumers, particularly of such adaptable pantiliners that are designed for everyday use with a variety of different panties and are intended to provide a "barely there" feel for users.

### Stiffness

The stiffness of the core-free area of an absorbent article is measured using a Taber Stiffness Tester (model 150-B, Taber Industries, North Tonawanda, N.Y.), equipped with a high sensitivity attachment (SR, Sensitivity Range) and a 10 unit compensator (both available from Taber Industries). For this setup, a 15 degree deflection to the right is used to measure each test specimen in the machine direction. The system is calibrated and operated per the manufacturer's instructions. All measurements are performed in a laboratory maintained at 23 °C ± 2 C° and 50% ± 2% relative humidity and test samples are conditioned in this environment for at least 2 hours prior to testing.

To prepare a test specimen, first obtain a test sample and remove it from any wrapper present. If folded, gently unfold it and smooth out any wrinkles. If present, remove the release paper to expose the adhesive on the garment facing side of the test sample. Lightly apply talc powder to the adhesive on the backsheet to mitigate tackiness. Obtain a test specimen by removing it from the core-free area (depicted as 18 in Figure 1) of the test sample, in this case, in the rear region of the test sample. When excising the test specimen from a test sample, use care to not impart any contamination or distortion to the test specimen during the process. The test specimen is obtained from an area free of folds or wrinkles. An ideal test specimen has a 3.81 cm width (parallel to the transverse axis) and a 3.81 cm length (parallel to the longitudinal axis of the test sample). To accommodate a small core free region, a smaller test specimen can be used, however it must have a length of 3.81 cm. The test specimen does not have to be excised from an area that is parallel to the longitudinal axis of the test sample, but it must be rectangular in shape (e.g. no curved edges) with a width as large as the core-free region will allow. Calculate the area of the test specimen and record to the nearest 0.1 square centimeter. Test results for any test specimen that is less than the ideal size will be normalized based on the area tested.

Orient the test specimen such that the topsheet side is facing up. Insert the test specimen into the high sensitivity attachment such that the second and fourth pins from the left are above the test specimen, the first and third pins from the left are below the test specimen, and the length of the test specimen is perpendicular to the pins. Ensure that the pins make contact with the test specimen but do not exert pressure on it. Measure the test specimen with a 15 degree deflection to the right and record the result to the nearest 1 Taber Stiffness Unit. Multiply the result by 0.01 (scaling multiplier for the SR Attachment + 10 Unit Compensator) and record to the nearest 0.01 Taber Stiffness Unit. Normalize the result for any test specimen that was less than 3.81 cm x 3.81 cm by dividing the result (in Taber Stiffness Units) by the specimen area (in square centimeters) and then multiply by 14.5 square centimeters (area of ideal sample size). Note that 1 Taber Stiffness Unit is equivalent to 1 gram centimeter. Report results to the nearest 0.01 g*cm as Core Free Area Stiffness.

In like fashion, repeat for a total of five replicate test specimens obtained from five substantially similar test samples. Calculate the arithmetic mean for Core Free Area Stiffness and report to the nearest 0.01 g*cm.

The stiffness of the core-containing region of an absorbent article is measured using a Taber Stiffness Tester (model 150-B, Taber Industries, North Tonawanda, N.Y.), equipped to measure test specimens in the 1 - 10 test range (requires a 10 unit compensator, available from Taber Industries). For this setup, a 15 degree deflection to the left and right is used to measure each test specimen in the machine direction. The system is calibrated and operated per the manufacturer's instructions. All measurements are performed in a laboratory maintained at 23 °C ± 2 C° and 50% ± 2% relative humidity and test samples are conditioned in this environment for at least 2 hours prior to testing.

To prepare a test specimen, first obtain a test sample and remove it from any wrapper present. If folded, gently unfold it and smooth out any wrinkles. If present, remove the release paper to expose the adhesive on the garment facing side of the test sample. Lightly apply talc powder to the adhesive on the backsheet to mitigate tackiness. Obtain a test specimen by excising it from the test region of the test sample in an area free of folds or wrinkles. The test region is preferably at the intersection of the longitudinal and transverse midpoints of the primary absorbent core, however the test specimen must contain the largest sampling of the primary absorbent core as possible. The edges of the test specimen must be parallel to the longitudinal and transverse axes of the test sample. When excising the test specimen from a test sample, use care to not impart any contamination or distortion to the test specimen during the process. An ideal test specimen has a 3.81 cm width (parallel to the transverse axis of the test sample) and a 3.81 cm length (parallel to the longitudinal axis of the test sample). To accommodate a narrow test region, a smaller test specimen can be used, however it must have a length of 3.81 cm and be rectangular in shape (e.g. no curved edges). If the test specimen is smaller than the ideal size, calculate the area of the test specimen and record to the nearest 0.1 square centimeter. Test results for any test specimen that is less than the ideal size will be normalized based on the area tested.

Following the manufacturer's instructions, set up the Taber Stiffness Tester to obtain measurements in the 0 - 10 range. In this setup, the rollers are mounted on the unit in the up position and the 10 unit compensator is mounted on the pendulum. Insert the test specimen into the clamp jaws with the length of the test specimen vertically aligned. Ensure the bottom edge of the test specimen is resting on the bottom gauge that lies beneath the rollers. Using the clamp screws, tighten the test specimen between the clamp jaws such that it is centered horizontally and vertically in the fixture. Now use the roller adjustment knobs to center the test specimen between the rollers ensuring that the pendulum is not deflected in the process. Measure the test specimen with a 15 degree deflection to the left, then to the right, and record each result to the nearest 1 Taber Stiffness Unit. Calculate the average of the left and right results, then multiply the average by 0.1 (scaling multiplier for the 10 Unit Compensator) and record to the nearest 0.01 Taber Stiffness Unit. Normalize the result for any test specimen that was less than 3.81 cm x 3.81 cm by dividing the result (in Taber Stiffness Units) by the specimen area (in square centimeters) and then multiply by 14.5 square centimeters (area of ideal sample size). Note that 1 Taber Stiffness Unit is equivalent to 1 gram centimeter. Report results as Core Area Stiffness to the nearest 0.01 g*cm.

In like fashion, repeat for a total of five replicate test specimens obtained from five substantially similar test samples. Calculate the arithmetic mean for Core Area Stiffness and report to the nearest 0.01 g*cm.

**Table 2a:**

| **CORE STIFFNESS (g*cm)** | Comparative Sample 1 | Inventive Sample 1 |
|---|---|---|
| Core | 4.15 | 2.51 |
| Core Free Area | 0.47 | 0.10 |

### In-Use Flexibility

The bending properties of an absorbent article in adapted form are measured on a constant rate of extension tensile tester (a suitable instrument is the MTS Alliance using Testworks 4.0 Software, as available from MTS Systems Corp., Eden Prairie, MN) using a load cell for which the forces measured are within 1% to 99% of the limit of the cell. All testing is performed in a room controlled at 23°C ± 3C° and 50% ± 2% relative humidity and test samples are conditioned in this environment for at least 2 hours prior to testing.

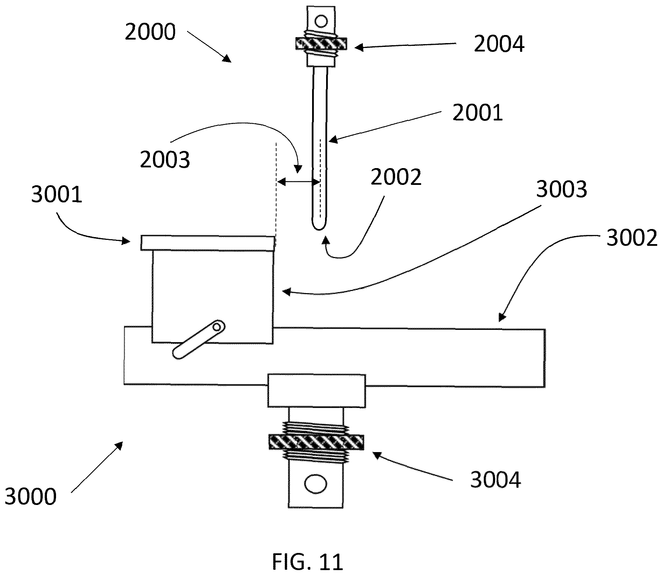

The bending fixture used for this test is depicted in Figure 11. The bottom stationary fixture 3000 consists of a horizontal support platform 3001 that has squared edges and is mounted on a rail 3002 which allows it to be moved horizontally left to right and locked into position. The bottom fixture 3000 is constructed such that it has an adequate vertical clearance between the top surface of the platform 3001 and the horizontal rail 3002 to prevent contact between the test sample and any underlying surface during testing. The support platform 3001 is about 10 mm thick and is made of polished stainless steel. It has a width greater than the width of the test sample and a length that is adequate to support the test sample. The edge of the support platform 3001 that is positioned closest to and directly adjacent to the blade 2001 is known as the leading edge 3003. The top movable fixture 2000 consists of a blade 2001 that is 3.0 mm thick and at least 40 mm tall, with a curved bottom edge 2002 (1.50 mm radius of curvature). The width of the blade 2001 must be greater than the width of the test sample. The blade 2001 is made of a light weight material that will enable maximizing the load cell capacity. The bottom 3000 and top 2000 fixtures both include an integral adapter 3004, 2004 appropriate to fit the respective position on the tensile tester frame and lock into position such that the blade 2001 is orthogonal to the motion of the crossbeam of the tensile tester.

Prior to testing, the test sample is applied to a piece of standard cotton fabric cut into a triangular shape. The fabric is 100% bleached cotton weave, about 100 g/m² (Style #429W) and is available from Testfabrics, Inc., 415 Delaware Avenue, West Pittston, PA 18643. Additional distributors of this fabric can be found on the Testfabrics website, www.testfabrics.com. Cut a piece of the cotton fabric into a triangle with a base of 60.0 mm and a height of 200.0 mm. Mark a line that is parallel to the base and 50.0 mm down from the apex of the triangle to denote the Sample Edge Line. A separate cotton triangle is required for each replicate.

To prepare the test sample, first remove it from any wrapper present. If folded, gently unfold it, smooth out any wrinkles, and determine which end of the test sample is intended to be the rear of the product (e.g. might contain a narrow core area). If the absorbent core of the test sample is symmetrical along the transverse midpoint, then simply assign one end to be the rear. Place the test sample onto a horizontally flat surface with the topsheet side facing down. If present, remove the release paper to expose the adhesive on the garment facing side of the test sample. Attach the prepared cotton triangle to the adhesive side of the test sample as follows. Ensure that the longitudinal axes of the test sample and cotton triangle are aligned. Then gently place the cotton triangle onto the test sample, with the Sample Edge Line on the triangle aligned with the rear edge of the test sample. Now fold the core-free regions (depicted as 18 in Figure 1) at the rear of the test sample over the cotton triangle, conforming the test sample to the triangle edges when folding, and secure using the adhesive. If there is no pronounced core-free region at the rear of the test sample, then simply fold the lateral edges along the rear portion of the test sample over the cotton triangle, conforming the test sample to the triangle edges when folding. If the test sample does not have an adhesive applied to its garment facing side, apply a light coating of adhesive to the garment facing side of the test sample using an aerosol glue (such as Elmer's Multipurpose Spray Adhesive, available from any convenient source, or equivalent). Then proceed with attaching the cotton triangle to the test sample as described above. Once the test sample is attached and folded around the cotton triangle, trim the cotton fabric that extends beyond the rear edge of the test sample flush along the rear edge of the test sample. On the garment facing side of the prepared test sample, draw a line that is parallel to the transverse axis of the test sample and 30.0 mm from the rear edge (narrow end) to denote the Test Location. Lay the prepared test sample horizontally flat on a rigid surface, then apply a 5 pound weight (base dimensions about 38 mm by 149 mm) over the test sample for at least 5 minutes to ensure attachment between the test sample and the cotton triangle. Use care when handling the prepared test sample to ensure it is not bent or twisted prior to testing.

Prepare the tensile tester as follows. Position the platform 3001 on its rail 3002 so that it is under the blade 2001. Move the blade 2001 down until it is in contact with the platform 3001. Make the necessary adjustments to ensure the bottom surface of the blade is parallel to the surface of the platform 3001. Now move the platform 3001 to the left of the blade 2001. The blade 2001 is then lowered, and the position of the platform 3001 is adjusted on its rail so that its leading edge 3003 is in contact with the blade 2001. Make the necessary adjustments to ensure that the blade 2001 and leading edge 3003 are parallel and aligned front to back with no skew. Now adjust the position of the platform 3001 such that the distance between its leading edge 3003 and the center of the curved bottom edge 2002 of the blade 2001 is 30.0 ± 0.5 mm, and lock it in place. Raise the blade 2001 about 15 mm so that it does not interfere with the mounting of the test sample. Program the tensile tester for a compression test to lower the crosshead at an initial rate of 5 cm per minute until the blade 2001 contacts the sample with 10 mN of force. Zero the crosshead. Lower the blade 2001 at a rate of 50.0 cm per minute with a displacement of 20.0 mm and collect force (mN) and displacement (mm) data at 400 Hz throughout the test. Then return the crosshead to its original location.

Place the test sample, with the topsheet side facing down, onto the top surface of the support platform 3001. Extend the rear edge of the test sample over the leading edge 3003 of the support platform 3001 and position it such that the Test Location is parallel to the leading edge 3003 and laterally centered under the blade 2001. Place a weight over the portion of the test sample that remains on the surface of the support platform 3001 to hold it in place during testing. The weight is heavy enough to secure the test sample and is positioned with its front edge flush with the leading edge 3003 of the support platform 3001. Zero the load cell and start the test. Construct a graph of force (mN) versus displacement (mm). Read the maximum peak force (mN) from the graph and record as Pad Flexibility to the nearest 0.1 mN.

In like fashion, repeat the entire test for a total of five substantially similar test samples. Calculate the arithmetic mean for the peak force and report as Whole Product Flexibility to the nearest 0.1 mN.

**Table 2b:**

| In-Use Flexibility (mN) | Comparative Sample 1 | Inventive Sample 1 |
|---|---|---|
| | 177.5 | 110.2 |

It can be seen in Tables 2a and 2b that the pantiliner described herein is less stiff and overall more flexible in the core region than comparable products, while still carrying the same/similar or more capacity (as shown in Table 4 below). The lower stiffness level allows the pantiliner to conform and adapt to a user's position, even in the front region of the pantiliner where the majority of the core is located. Furthermore, the pantiliner described herein is also less stiff than a comparable adaptable product (comparative sample 1) in the core-free area. This is the region of the pantiliner that is intended to be adaptable for use with different panties. The reduced stiffness in this region enables the pantiliner described herein to be folded in a variety of different configurations dependent on the needs of the specific panties with which it is being worn (as shown in Figures 7A to 7D). By comparison, as the comparative sample 1 is significantly stiffer in this core free area, it is provided with so-called folding lines (described in EP 1757257 B1) to facilitate folding of the core free area along the specific fold line. Thus, the pantiliner described herein is more adaptable than those currently commercially available.

### Chassis Extensibility

The tensile properties of the core-free area of an absorbent article are measured on a constant rate of extension tensile tester (a suitable instrument is the MTS Alliance using Testworks 4.0 Software, as available from MTS Systems Corp., Eden Prairie, MN) using a load cell for which the forces measured are within 1% to 99% of the limit of the cell. All testing is performed in a room controlled at 23°C ± 3C° and 50% ± 2% relative humidity and test samples are conditioned in this environment for at least 2 hours prior to testing.

To hold the test specimen, an identical set of grips are used, with one attached to the bottom fixture and the other attached to the top fixture of the tensile tester. The grips are manual and lightweight to maximize the capacity of the load cell, and they must be wider than the test specimen. The bar grips are constructed in such a way that a single line of gripping force along a line perpendicular to the pull axis of the tensile tester is enabled. The top and bottom grips are mounted in such a way that they are horizontally and vertically aligned.

To prepare a test specimen, first obtain a test sample and remove it from any wrapper present. If folded, gently unfold it and smooth out any wrinkles. If present, remove the release paper to expose the adhesive on the garment facing side of the test sample. Lightly apply talc powder to the adhesive on the backsheet to mitigate tackiness. Obtain a test specimen that is at least 31.0 mm long such that the length enables a gauge length of 25.0 mm (parallel to the longitudinal axis of the absorbent article) at a width of 10.0 mm (parallel to the transverse axis of the absorbent article). The test specimen is to be excised from the core-free region of the test sample in an area free of folds or wrinkles. The core-free region (depicted as 18 in Figure 1) is the area near the rear of the test sample that does not contain the primary absorbent core. When excising the test specimen from a test sample, use care to not impart any contamination or distortion to the test specimen during the process.

Program the tensile tester for a constant rate of extension uniaxial elongation to break test as follows. Set the gauge length to 25.0 mm (Lᵢₙᵢₜᵢₐₗ) using a calibrated gauge block and zero the crosshead. Insert the test specimen into the grips such that the long side is centered and parallel to the central pull axis of the tensile tester. Raise the crosshead at a rate of 1.00 mm/s until the test specimen breaks, collecting force (N) and extension (mm) data at 100 Hz throughout the test. Return the crosshead to its original location. Construct a graph of force (N) versus extension (mm). Read the maximum peak force (N) from the graph and record as Peak Force to the nearest 0.1 N. Read the extension (mm) at the maximum Peak Force from the graph and record as Lₚₑₐₖ to the nearest 0.1 mm. Calculate Elongation at Peak as [ (Lₚₑₐₖ / Lᵢₙᵢₜᵢₐₗ) * 100 ] and record to the nearest 0.1 %.

In like fashion, repeat the test for a total of five replicate test specimens obtained from five substantially similar test samples. Calculate the arithmetic mean for Peak Force and report to the nearest 0.1 N. Calculate the arithmetic mean for Elongation at Peak and report to the nearest 0.1 %.

**Table 3:**

| **Product** | **N** | **Peak Extension Force (N)** | **Elongation @ Peak (%)** |
|---|---|---|---|
| Comparative Example 1 | 1 | 29.82 | 54.4 |
| | 2 | 29.38 | 44.4 |
| | 3 | 29.19 | 44.3 |
| | 4 | 30.64 | 52.3 |
| | 5 | 31.92 | 49.7 |
| Average | | **30.19** | **49.0** |
| Std. Dev | | 1.115 | 4.6 |
| Inventive Example 1 | 1 | 13.29 | 91.2 |
| | 2 | 11.88 | 79.4 |
| | 3 | 10.39 | 75.5 |
| | 4 | 13.16 | 93.7 |
| | 5 | 13.31 | 97.5 |
| Average | | **12.41** | **87.5** |
| Std. Dev | | 1.274 | 9.5 |

From Table 3, it can be seen that the elongation prior to breakage in the longitudinal direction of the pantiliner described herein is nearly double that of the comparable adaptable pantiliner. Thus, during use, the pantiliner described herein conforms much better with regular movements of a user. Furthermore, the force required to extend the pantiliner in the longitudinal direction is less, thus the pantiliner will respond to even small movements.

### Absorbent Capacity - Dunk Test Method

The dunk test determines the theoretical absorbent capacity of an absorbent article. The test sample is soaked in a test fluid for a prescribed amount of time, then subjected to a static weight on an inclined platform. The amount of test fluid that is retained by the test sample is its Absorbent Capacity. All testing is performed in a room controlled at 23°C ± 3C° and 50% ± 2% relative humidity and test samples are conditioned in this environment for at least 2 hours prior to testing.

The equipment needed to perform this test includes a liquid reservoir for soaking the test sample and an inclined platform and weight assembly for draining the excess test fluid. The liquid reservoir is a shallow dish that is large enough to allow the entire test sample to lie horizontally flat inside, and deep enough to keep the test sample fully submerged in the test fluid. The inclined platform is made of Plexiglass (or equivalent) and has a 15 degree angle to the horizontal. The inclined platform is large enough to support the entire test sample and weight assembly. One of two different weight assemblies is used depending on the length of the test sample. For test samples with a longitudinal length less than or equal to 15.5 cm, the weight assembly has a mass of 1320 g ± 15 g with base dimensions of 15.5 cm by 5 cm. For test samples with a longitudinal length greater than 15.5 cm, the weight assembly has a mass of 2265 g ± 15 g with base dimensions of 20.5 cm by 6.5 cm. The weight assembly is foam-padded and constructed as follows. Lay a piece of polyethylene film (any convenient source) flat on a bench surface. A piece of polyurethane foam (25 mm thick; base dimensions determined by test sample length as previously stated; available from Concord-Renn Co. Cincinnati, OH, density of 1.0 lb/ft³, IDL 24 psi) is laid centered on top of the film. A piece of Plexiglas (6.4 mm thick; base dimensions determined by test sample length as previously stated) is then stacked on top of the polyurethane foam. Next the polyethylene film is used to wrap the polyurethane foam and Plexiglas plate securing it with transparent tape. A metal weight with handle is stacked on top of, and fastened to, the Plexiglass plate. In order to prevent the weight assembly from sliding down the inclined platform during the test, a support arm fixed to a ring stand with a non-slip base is used to keep the weight assembly in place.

The reagent grade components needed for the test fluid preparation are all available from VWR International (or an equivalent source) and include: Urea (NH₂CONH₂, CAS No 57-13-6), Sodium Chloride (NaCl, CAS No 7647-14-5), Magnesium Sulfate Heptahydrate (MgSO₄ 7H₂O, CAS No 10034-99-8), Calcium Chloride anhydrous (CaCl2, CAS No 10043-52-4) and deionized water. To prepare the test fluid, add 2.0% w/w Urea, 0.90% w/w Sodium Chloride, 0.11% w/w Magnesium Sulfate Heptahydrate, 0.06% w/w Calcium Chloride anhydrous to 96.93% w/w deionized water in a beaker of sufficient size and mix thoroughly.

To prepare the test sample, first remove it from any wrapper present. If folded, gently unfold it and smooth out any wrinkles. If present, leave the release paper in place. Obtain the mass of the test sample (including release paper if applicable) and record as Dry Mass to the nearest 0.01 g.

Fill the liquid reservoir with a sufficient amount of test fluid to keep the test sample submerged at least 5 mm beneath the surface of the test fluid for the entire soak time. With the topsheet side of the test sample facing the test fluid, submerge the test sample into the test fluid and allow it to soak for 25.0 minutes. After 25.0 minutes has elapsed, grip the test sample by one longitudinal edge, remove it from the test fluid and allow it to drip in a vertical position for 2.0 seconds. Using care not to bend or twist the test sample, transfer it to the inclined platform with the topsheet side facing the platform surface. While holding the test sample in place at one longitudinal end, gently place the weight assembly onto the test sample, ensuring that it is centered over the absorbent core of the test sample with the longitudinal axes of both are aligned. Use the support arm to ensure the weight does not slide down the incline. The weight assembly is applied to the test sample for 2.0 minutes. After 2.0 minutes, gently remove the weight assembly. Grip the test sample by one longitudinal edge and allow it to drip in a vertical position for 2.0 seconds, ensuring the test sample is not bent or twisted in the process. Obtain the mass of the wetted test sample and record as Wet Mass to the nearest 0.01 g. Calculate the amount of liquid retained by the test sample by subtracting the Dry Mass from the Wet Mass, and record as Absorbent Capacity to the nearest 0.01 g.

In like fashion, repeat the test for a total of five substantially similar test samples. Calculate the arithmetic mean for Absorbent Capacity and report to the nearest 0.01 g.

**Table 4:**

| **Absorbent Capacity (g)** | Comparative Sample 1 | Inventive Sample 1 |
|---|---|---|
| Whole Product | 11.65 | 10.56 |

It can be seen in Table 4 that the pantiliner described herein has a similar absorbent capacity to comparative sample 1 despite being considerably less thick and stiff.

## Claims

1. An absorbent article having a longitudinal axis and a transverse axis, the article comprising:
a topsheet;
a backsheet, wherein the topsheet and the backsheet together form a chassis of the absorbent article, said chassis being symmetrical about the transverse axis;
an absorbent core disposed between the topsheet and the backsheet, wherein a perimeter of the chassis extends beyond the surface area of the absorbent core, and wherein the absorbent core is asymmetrical about the transverse axis;
wherein less than 50% of the surface area of the absorbent article is mechanically compressed;
wherein the absorbent article has an in-use flexibility of less than 170mN, preferably less than 130 mN as measured by the in-use flexibility test described herein.

2. An absorbent article as claimed in claim 1, wherein the absorbent article has a stiffness in the core area of between 0.5 g*cm and less than 4.0 g*cm as measured in accordance with the Stiffness test method described herein.

3. An absorbent article as claimed in any preceding claim, wherein the absorbent article has a stiffness measured in the core free area of between 0.01 g*cm and 0.4 g*cm as measured in accordance with the Stiffness test method described herein.

4. An absorbent article as claimed in any preceding claim, wherein the core free area has a peak extension force of less than 25 N when measured in the longitudinal direction.

5. An absorbent article as claimed in any preceding claim, wherein the absorbent article has a thickness measured in the core area of between 0.5 and 2.0 mm.

6. An absorbent article as claimed in any preceding claim, wherein the absorbent article has a thickness measured in the core free area of between 0.1 mm and 0.7 mm.

7. An absorbent article as claimed in any preceding claim, wherein there is substantially no crimping around the perimeter of the absorbent article.

8. An absorbent article as claimed in any preceding claim, wherein less than 10% of the core-free area is mechanically compressed.

9. An absorbent article as claimed in any preceding claim, wherein between 10% and 50% of the surface area of the absorbent core is embossed.

10. An absorbent article as claimed in claim 9, wherein the embossed pattern comprises a series of fine lines intersecting one another.

11. An absorbent article as claimed in any preceding claim, wherein more than 50% of the absorbent core is disposed in a front region of the absorbent article.

12. An absorbent article as claimed in any preceding claim, wherein the absorbent article has an absorbent capacity of at least 3g and less than 20g.

13. An absorbent article as claimed in any preceding claim, wherein the topsheet is secured to the backsheet around the perimeter of the absorbent core with adhesive only.

14. An absorbent article as claimed in any preceding claim, wherein the topsheet is a laminated topsheet. and the primary and secondary topsheet are secured together with adhesive only.

15. An absorbent article as claimed in any preceding claim, wherein the laminated topsheet is mechanically compressed only in the core region of the absorbent article.

16. An absorbent article as claimed in claim 14 or 15, wherein the laminated topsheet comprises a spunbond nonwoven primary topsheet and an air carded nonwoven secondary topsheet.

## Patentansprüche

1. Absorptionsartikel, der eine Längsachse und eine Querachse aufweist, der Artikel umfassend:
eine Oberschicht;
eine Unterschicht, wobei die Oberschicht und die Unterschicht zusammen eine Grundeinheit des Absorptionsartikels ausbilden, wobei die Grundeinheit um die Querachse symmetrisch ist;
einen Absorptionskern, der zwischen der Oberschicht und der Unterschicht angeordnet ist, wobei sich ein Umfang der Grundeinheit über den Oberflächenbereich des Absorptionskerns hinaus erstreckt und wobei der Absorptionskern um die Querachse asymmetrisch ist;
wobei weniger als 50 % des Oberflächenbereichs des Absorptionsartikels mechanisch komprimiert sind;
wobei der Absorptionsartikel eine Gebrauchsflexibilität von weniger als 170 mN, vorzugsweise weniger als 130 mN, aufweist, wie durch den hierin beschriebenen Gebrauchsflexibilitätstest gemessen.

2. Absorptionsartikel nach Anspruch 1, wobei der Absorptionsartikel eine Steifheit in dem Kernbereich zwischen 0,5 g*cm und weniger als 4,0 g*cm aufweist, wie gemäß dem hierin beschriebenen Steifheitsprüfverfahren gemessen.

3. Absorptionsartikel nach einem der vorstehenden Ansprüche, wobei der Absorptionsartikel eine Steifheit in dem kernfreien Bereich zwischen 0,01 g*cm und 0,4 g*cm aufweist, wie gemäß dem hierin beschriebenen Steifheitsprüfverfahren gemessen.

4. Absorptionsartikel nach einem der vorstehenden Ansprüche, wobei der kernfreie Bereich eine Spitzendehnkraft von weniger als 25 N aufweist, wenn in der Längsrichtung gemessen.

5. Absorptionsartikel nach einem der vorstehenden Ansprüche, wobei der Absorptionsartikel eine Dicke, die in dem Kernbereich gemessen wird, zwischen 0,5 und 2,0 mm aufweist.

6. Absorptionsartikel nach einem der vorstehenden Ansprüche, wobei der Absorptionsartikel eine Dicke, die in dem kernfreien Bereich gemessen wird, zwischen 0,1 mm und 0,7 mm aufweist.

7. Absorptionsartikel nach einem der vorstehenden Ansprüche, wobei im Wesentlichen kein Kräuseln um den Umfang des Absorptionsartikels herum vorhanden ist.

8. Absorptionsartikel nach einem der vorstehenden Ansprüche, wobei weniger als 10 % des kernfreien Bereichs mechanisch komprimiert sind.

9. Absorptionsartikel nach einem der vorstehenden Ansprüche, wobei zwischen 10 % und 50 % des Oberflächenbereichs des Absorptionskerns mit Prägungen versehen sind.

10. Absorptionsartikel nach Anspruch 9, wobei das Prägemuster eine Reihe feine, sich schneidende Linien umfasst.

11. Absorptionsartikel nach einem der vorstehenden Ansprüche, wobei mehr als 50 % des Absorptionskerns in einer vorderen Region des Absorptionskerns angeordnet sind.

12. Absorptionsartikel nach einem der vorstehenden Ansprüche, wobei der Absorptionsartikel ein Absorptionsvermögen von mindestens 3 g und weniger als 20 g aufweist.

13. Absorptionsartikel nach einem der vorstehenden Ansprüche, wobei die Oberschicht an der Unterschicht um den Umfang des Absorptionskerns herum nur mit Klebstoff befestigt ist.

14. Absorptionsartikel nach einem der vorstehenden Ansprüche, wobei die Oberschicht eine laminierte Oberschicht ist und die primäre und sekundäre Oberschicht nur mit Klebstoff aneinander befestigt sind.

15. Absorptionsartikel nach einem der vorstehenden Ansprüche, wobei die laminierte Oberschicht nur in dem Kernbereich des Absorptionsartikels mechanisch komprimiert ist.

16. Absorptionsartikel nach Anspruch 14 oder 15, wobei die laminierte Oberschicht eine primäre Oberschicht aus Spinnvlies und eine sekundäre Oberschicht aus luftkardiertem Vlies umfasst.

## Revendications

1. Article absorbant (20) ayant un axe longitudinal et un axe transversal, l'article comprenant :
une feuille supérieure ;
une feuille arrière, dans lequel la feuille supérieure et la feuille arrière forment ensemble un châssis de l'article absorbant, ledit châssis étant symétrique par rapport à l'axe transversal ;
un noyau absorbant disposé entre la feuille supérieure et la feuille arrière, dans lequel un périmètre du châssis s'étend au-delà de la surface du noyau absorbant, et dans lequel le noyau absorbant est asymétrique par rapport à l'axe transversal ;
dans lequel moins de 50 % de la surface de l'article absorbant est comprimée mécaniquement ;
dans lequel l'article absorbant a une souplesse en cours d'utilisation inférieure à 170 mN, de préférence inférieure à 130 mN, telle que mesurée par le test de souplesse en cours d'utilisation décrit dans la description.

2. Article absorbant selon la revendication 1, dans lequel l'article absorbant a une rigidité dans la zone centrale comprise entre 0,5 g*cm et moins de 4,0 g*cm, mesurée conformément au procédé de test de rigidité décrite dans la description.

3. Article absorbant selon l'une quelconque revendication précédente, dans lequel l'article absorbant a une rigidité mesurée dans la zone libre du noyau comprise entre 0,01 g*cm et 0,4 g*cm, mesurée conformément au procédé de test de rigidité décrite dans la description.

4. Article absorbant selon l'une quelconque revendication précédente, dans lequel la zone libre du noyau a une force d'extension maximale inférieure à 25 N lorsqu'elle est mesurée dans le sens longitudinal.

5. Article absorbant selon l'une quelconque revendication précédente, dans lequel l'article absorbant a une épaisseur mesurée dans la zone centrale comprise entre 0,5 et 2,0 mm.

6. Article absorbant selon l'une quelconque revendication précédente, dans lequel l'article absorbant a une épaisseur mesurée dans la zone libre du noyau comprise entre 0,1 mm et 0,7 mm.

7. Article absorbant selon l'une quelconque revendication précédente, dans lequel il n'y a essentiellement pas de sertissage autour du périmètre de l'article absorbant.

8. Article absorbant selon l'une quelconque revendication précédente, dans lequel moins de 10 % de la zone sans noyau est comprimée mécaniquement.

9. Article absorbant selon l'une quelconque revendication précédente, dans lequel entre 10 % et 50 % de la surface du noyau absorbant est gaufrée.

10. Article absorbant selon la revendication 9, dans lequel le motif gaufré comprend une série de lignes fines se croisant les unes les autres.

11. Article absorbant selon l'une quelconque revendication précédente, dans lequel plus de 50 % du noyau absorbant est disposé dans une région avant de l'article absorbant.

12. Article absorbant selon l'une quelconque revendication précédente, dans lequel l'article absorbant a une capacité absorbante d'au moins 3 g et de moins de 20 g.

13. Article absorbant selon l'une quelconque revendication précédente, dans lequel la feuille supérieure est fixée à la feuille arrière sur le périmètre du noyau absorbant, uniquement avec de l'adhésif.

14. Article absorbant selon l'une quelconque revendication précédente, dans lequel la feuille supérieure est une feuille supérieure stratifiée et les feuilles supérieures primaire et secondaire sont fixées ensemble uniquement avec de l'adhésif.

15. Article absorbant selon l'une quelconque revendication précédente, dans lequel la feuille supérieure laminée est comprimée mécaniquement uniquement dans la région centrale de l'article absorbant.

16. Article absorbant selon la revendication 14 ou 15, dans lequel la feuille supérieure laminée comprend une feuille supérieure primaire non tissée filée-liée et une feuille supérieure secondaire non tissée cardée à l'air.
